# FASCICULE DE BREVET EUROPEEN

(11) **EP 3 017 069 B1**
(45) Date de publication et mention de la délivrance du brevet: **16.01.2019**
(21) Numéro de dépôt: 14725738.0
(22) Date de dépôt: 23.05.2014
(51) Int. Cl.: C07K 7/56, C07K 7/64, C12R 1/01, C11D 3/48, C11D 3/28, C11D 3/32, C11D 3/38, A61K 35/00, A61K 35/74, A61K 35/741, A61K 38/00, A61K 38/12, A23K 10/18, A23K 20/195, A23K 50/80

(54) **UTILISATION D'UNE BACTÉRIE ISOLÉE DU GENRE PSEUDOALTEROMONAS, CYCLOLIPOPEPTIDES ET LEURS UTILISATIONS**
VERWENDUNG EINES AUS DER GATTUNG PSEUDOALTEROMONAS ISOLIERTEN BAKTERIUMS, CYCLOLIPOPEPTIDE UND VERWENDUNGEN DAVON
USE OF A BACTERIUM ISOLATED FROM THE GENUS PSEUDOALTEROMONAS, CYCLOLIPOPEPTIDES AND USES THEREOF

(30) Priorité: 24.05.2013 FR 1354691
(43) Date de publication de la demande: 11.05.2016
(73) Titulaire: Centre National de la Recherche Scientifique (C.N.R.S.), 75016 Paris (FR); Université de Rennes 1, 35065 Rennes Cedex (FR); Université de Bretagne Occidentale, 29200 Brest (FR)
(72) Inventeur: DESRIAC, Florie, F-29380 Bannalec (FR); FLEURY, Yannick, F-29000 Quimper (FR); LE CHEVALIER, Patrick, F-29950 Clohars-fouesnant (FR); DESTOUMIEUX, Delphine, F-34380 Viols-le-fort (FR); SIMON, Mathieu, F-34000 Montpellier (FR)
(74) Mandataire: Lavoix
(86) Numéro de dépôt international: PCT/EP2014/060737
(87) Numéro de publication internationale: WO 2014/187993

(56) Documents cités:
- DIANE DEFER ET AL: "Antimicrobial peptides in oyster hemolymph: The bacterial connection", FISH & SHELLFISH IMMUNOLOGY, vol. 34, no. 6, 22 mars 2013 (2013-03-22), pages 1439-1447, XP055096146, ISSN: 1050-4648, DOI: 10.1016/j.fsi.2013.03.357 -& DATABASE EMBL [Online] 21 novembre 2012 (2012-11-21), "Pseudoalteromonas sp. hCg-6 16S ribosomal RNA gene, partial sequence.", XP002718723, extrait de EBI accession no. EM_STD:JX912482 Database accession no. JX912482
- EVGENIY S. BALAKIREV ET AL: "Symbiotic Associations in the Phenotypically-Diverse Brown Alga Saccharina japonica", PLOS ONE, vol. 7, no. 6, 20 juin 2012 (2012-06-20), page e39587, XP055096499, DOI: 10.1371/journal.pone.0039587 -& DATABASE EMBL [Online] février 2012 (2012-02), "Uncultured proteobacterium clone ncSPLO71 16S ribosomal RNA gene, partial sequence.", XP002718724, extrait de EBI accession no. EM_STD:JQ218661 Database accession no. JQ218661
- HEINDL H ET AL: "Phylogenetic diversity and antimicrobial activities of bryozoan-associated bacteria isolated from Mediterranean and Baltic Sea habitats", SYSTEMATIC AND APPLIED MICROBIOLOGY, URBAN & FISCHER, AMSTERDAM, NL, vol. 33, no. 2, mars 2010 (2010-03), pages 94-104, XP026964663, ISSN: 0723-2020 [extrait le 2010-02-13] -& DATABASE EMBL [Online] 26 mars 2010 (2010-03-26), "Pseudoalteromonas sp. B201 partial 16S rRNA gene, strain B201", XP002718725, extrait de EBI accession no. EM_STD:FN295770 Database accession no. FN295770
- M. WIETZ ET AL: "Wide Distribution of Closely Related, Antibiotic-Producing Arthrobacter Strains throughout the Arctic Ocean", APPLIED AND ENVIRONMENTAL MICROBIOLOGY, vol. 78, no. 6, 15 mars 2012 (2012-03-15), pages 2039-2042, XP055096498, ISSN: 0099-2240, DOI: 10.1128/AEM.07096-11 -& DATABASE EMBL [Online] 2 mai 2011 (2011-05-02), "Pseudoalteromonas sp. MB33 16S ribosomal RNA gene, partial sequence.", XP002718726, extrait de EBI accession no. EM_STD:JF706643 Database accession no. JF706643 -& DATABASE EMBL [Online] 2 mai 2011 (2011-05-02), "Pseudoalteromonas sp. MB205 16S ribosomal RNA gene, partial sequence.", XP002718727, extrait de EBI accession no. EM_STD:JF706645 Database accession no. JF706645 -& DATABASE EMBL [Online] 2 mai 2011 (2011-05-02), "Pseudoalteromonas sp. MB220 16S ribosomal RNA gene, partial sequence.", XP002718728, extrait de EBI accession no. EM_STD:JF706646 Datab
- NATALIYA I KALINOVSKAYA ET AL: "â Pseudoalteromonas januariaâ SUT 11 as the Source of Rare Lipodepsipeptides", CURRENT MICROBIOLOGY, SPRINGER-VERLAG, NE, vol. 56, no. 3, 8 janvier 2008 (2008-01-08), pages 199-207, XP019587423, ISSN: 1432-0991
- JOHN P. BOWMAN: "Bioactive Compound Synthetic Capacity and Ecological Significance of Marine Bacterial Genus Pseudoalteromonas", MARINE DRUGS, vol. 5, no. 4, 18 décembre 2007 (2007-12-18), pages 220-241, XP055096332, ISSN: 1660-3397, DOI: 10.3390/md504220
- FLORIE DESRIAC ET AL: "Bacteriocin as Weapons in the Marine Animal-Associated Bacteria Warfare: Inventory and Potential Applications as an Aquaculture Probiotic", MARINE DRUGS, vol. 8, no. 4, janvier 2010 (2010-01), pages 1153-1177, XP055096147, ISSN: 1660-3397, DOI: 10.3390/md8041153

## Description

La présente divulgation concerne une bactérie isolée du genre *Pseudoalteromonas* pour son utilisation en tant que probiotique, l'utilisation de ladite souche en tant qu'agent conservateur. La présente invention concerne des cyclolipopeptides susceptibles d'être obtenus à partir de cette bactérie, une composition comprenant au moins un tel cyclolipopeptide, ainsi que leurs utilisations.

L'aquaculture rassemble toutes les activités de production animale ou végétale en milieu aquatique. L'aquaculture se pratique en bord de mer, de rivières ou d'étangs. Certains systèmes de récifs artificiels ou dispositifs attracteurs et de concentration peuvent être assimilés à de l'aquaculture, dès lors qu'il existe une offre directe en nourriture ou en support (indirectement produite à partir de remontée d'eau chargée en minéraux par exemple). Elle concerne notamment les productions de poissons (pisciculture), de coquillages (conchyliculture), de crustacés (astaciculture et pénéiculture), d'ormeaux (halioculture), d'huîtres (ostréiculture) ou encore d'algues (algoculture).

L'aquaculture est l'une des réponses apportées à la surpêche et aux besoins croissants de poisson. En 2008, elle fournissait dans le monde 76,4 % des poissons d'eau douce, 68,2 % des poissons diadromes, 64,1 % des mollusques, 46,4 % des crustacés et 2,6 % des poissons d'eau de mer consommés par l'homme.

Elle est parfois utilisée pour d'autres motifs que la consommation alimentaire, par exemple en Europe via de nombreuses "stations piscicoles" construites de 1850 à 1870, ou au Japon pour réintroduire dans l'environnement les crevettes ou des ormeaux là où ces animaux ont été surexploités ou ont disparu pour d'autres causes (pollution...).

De nos jours, l'intensification des modes de production et les variations climatiques ont rendu vulnérable le développement de l'aquaculture et de l'ostréiculture en particulier.

L'utilisation de probiotiques issus d'animaux sauvages sains s'inscrit dans une stratégie de bioprotection naturelle des espèces aquacoles. Si les probiotiques sont largement utilisés en élevage, leur utilisation en aquaculture reste encore une nouveauté.

La mise au point d'une stratégie biologique et efficace de protection de ces espèces apparaît donc comme donc un atout indéniable pour les producteurs et les industriels de la nutrition animale.

Par ailleurs, le renouvellement des agents antimicrobiens est aujourd'hui une priorité de santé publique et vétérinaire. Leur utilisation excessive et inadaptée a conduit à la sélection de souches multirésistantes qui provoquent des impasses thérapeutiques (25 000 décès/an dans la CEE, OMS 2011).

Depuis 30 ans, aucune nouvelle famille d'antibiotique anti-Gram-négatif n'a été mise sur le marché. La recherche de nouveaux composés présentant des propriétés antimicrobiennes est donc primordiale et urgente.

Parallèlement, il existe également de nos jours un besoin important dans le domaine des traitements phytosanitaires d'identifier des produits alternatifs, qui ne sont pas nocifs pour l'environnement, mais qui demeurent des agents bactércides efficaces.

Dans le cadre de la présente invention, les inventeurs ont isolé une bactérie marine présentant des propriétés utiles dans le secteur de la santé animale, en particulier dans l'aquaculture.

Cette bactérie marine produit notamment des composés antibactériens que les inventeurs ont caractérisés au niveau structurel et fonctionnel.

Cette bactérie appartient au genre *Pseudoalteromonas.*

Le genre *Pseudoalteromonas* a été proposé en premier par Gauthier et al. en 1995 et est à ce jour composé de 39 espèces (Gauthier, G., Gauthier, M. & Christen, R. (1995). Phylogenetic Analysis of the Genera Alteromonas, Shewanella, and Moritella Using Genes Coding for Small-Subunit rRNA Sequences and Division of the Genus Alteromonas into Two Genera, Alteromonas (Emended) and Pseudoalteromonas gen. nov., and Proposai of Twelve New Species Combinations. Int J Syst Bacteriol 45, 755-761).

Defer et al. ("Antimicrobial peptides in oyster hemolymph: The bacterial connection", FISH & SHELLFISH IMMUNOLOGY, vol. 34, no. 6, 22 mars 2013, pages 1439-1447*)* décrit l'isolement de souches bactériennes démontrant des potentialités antibactériennes et la capacité de produire des peptides antimicrobien. La souche *hCg-6* affiliée au genre *Pseudoalteromonas* est mentionnée, de même que le dépôt de la séquence partielle de son ADNr16S.

Ainsi, les inventeurs de la présente demande ont isolé une bactérie *Pseudoalteromonas hCg-6,* capable de produire des composés antibactériens, en particulier des cyclolipopeptides comprenant un cycle heptapeptidique et une chaîne hydrocarbonée de 4 à 20 atomes de carbone, plus particulièrement encore des cyclolipopeptides de formule A : 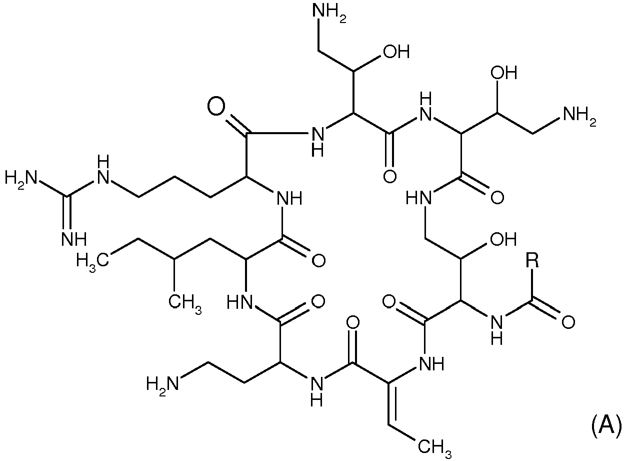 dans laquelle :
R est choisi dans le groupe constitué des groupes :
- alkyle comprenant entre 4 et 20 atomes de carbone, à l'exception des groupes nonyle et undécyle; ou
- alkylène comprenant entre 4 et 20 atomes de carbone, à l'exception des groupes undéc-4-ylène et tridéc-6-ylène; ou
- alkyle substitué par un groupement -OH et comprenant entre 4 et 20 atomes de carbone; et
- alkylène substitué par un groupement -OH et comprenant entre 4 et 20 atomes de carbone.

Plus particulièrement encore la bactérie isolée est la bactérie *Pseudoalteromonas hCg-6*, souche déposée selon le Traité de Budapest, le 22 mai 2013, auprès de la Collection Nationale de Culture de Microorganismes (CNCM, Paris, France) sous le numéro I-4753.

La souche *hCg-6* a été isolée de l'hémolymphe de l'huitre *Crassostrea* et a démontré qu'elle était capable de sécréter dans l'hémolymphe et le surnageant de différents milieux de culture des composés antibactériens.

L'étude des caractéristiques génétiques et biochimiques de cette souche ont permis de définir une nouvelle espèce, comme indiqué dans l'exemple qui suit.

Ladite bactérie est tout particulièrement intéressante dans la mesure où elle sécrète dans son surnageant de culture des composés antibactériens actifs contre des bactéries pathogènes, en particulier en aquaculture ou dans le domaine phytosanitaire.

Selon un de ces aspects, la présente divulgation concerne la bactérie isolée telle que mentionnée précédemment pour son utilisation en tant que probiotique pour l'élevage animal, en particulier pour l'aquaculture.

Plus particulièrement encore, la présente divulgation concerne la bactérie isolée telle que mentionnée ci-dessus pour son utilisation en tant que probiotique pour la pisciculture, la conchyliculture, l'halioculture, l'astaciculture, la pénéiculture, l'ostréiculture ou l'algoculture. Encore plus particulièrement, la présente divulgation concerne la bactérie isolée telle que mentionnée ci-dessus pour son utilisation pour améliorer la santé des poissons, des coquillages, des crustacés, des ormeaux, des huîtres ou encore des algues.

La présente divulgation concerne également l'utilisation de la bactérie isolée telle que mentionnée ci-dessus en tant que probiotique, en particulier pour la pisciculture, la conchyliculture, l'astaciculture, la pénéiculture, l'halioculture, l'ostréiculture ou l'algoculture. Encore plus particulièrement, la présente invention concerne l'utilisation de la bactérie isolée telle que mentionnée ci-dessus pour améliorer la santé des poissons, des coquillages, des crustacés, des ormeaux, des huîtres ou encore des algues.

La présente divulgation, selon un autre de ces aspects, concerne également une méthode de traitement des poissons, des coquillages, des crustacés, des ormeaux, des huîtres ou encore des algues, qui comprend l'administration d'une quantité efficace d'une bactérie selon l'invention. En particulier, cette méthode est destinée à améliorer la santé des poissons, des coquillages, des crustacés, des ormeaux, des huîtres ou encore des algues.

Par « probiotique », on entend un additif alimentaire comprenant une quantité efficace de la bactérie isolée selon l'invention, destiné à être introduit dans l'alimentation de l'animal. Selon l'OMS, les probiotiques sont des microorganismes vivants qui, administrés en quantité adéquate, confèrent à l'hôte un bénéfice sur sa santé (OMS, 2001).

Par « quantité efficace », on entend une quantité de bactérie qui permette la manifestation de l'effet recherché. En particulier, on entend une quantité comprise entre 10³ et 10¹⁰ UFC.mL⁻¹.

Selon un autre de ces aspects, la présente divulgation concerne également l'utilisation d'une bactérie telle que précédemment mentionnée, comme agent phytosanitaire.

La présente divulgation, selon un autre de ces aspects, concerne également une méthode de traitement phytosanitaire, qui comprend l'administration d'une quantité efficace d'une bactérie selon l'invention, en particulier à une plante, un champignon ou un insecte.

Par ailleurs, les inventeurs ont isolé une famille originale de cyclolipopeptides. Les cyclolipopeptides selon l'invention comprennent un cycle heptapeptidique acylé par une chaîne hydrocarbonée variant en longueur, en niveau d'insaturation et en hydroxylation.

Ainsi la présente invention concerne un cyclolipopeptide de formule A : 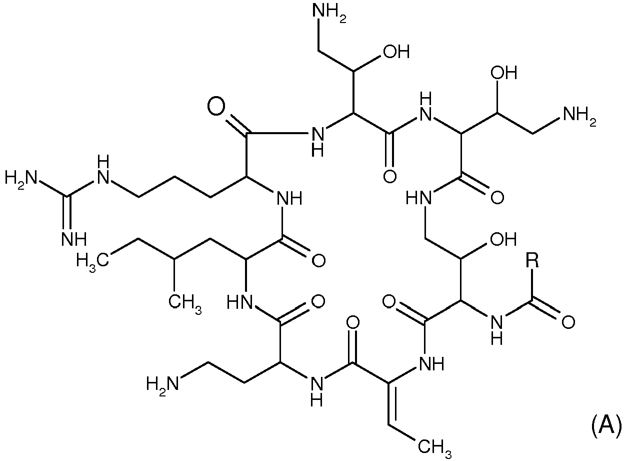 dans laquelle :
R est choisi dans le groupe constitué des groupes :
- alkyle comprenant entre 4 et 20 atomes de carbone, à l'exception des groupes nonyle et undécyle; ou
- alkylène comprenant entre 4 et 20 atomes de carbone, à l'exception des groupes undéc-4-ylène et tridéc-6-ylène; ou
- alkyle substitué par un groupement -OH et comprenant entre 4 et 20 atomes de carbone; et
- alkylène substitué par un groupement -OH et comprenant entre 4 et 20 atomes de carbone.

Par groupe « alkyle », on entend un groupe aliphatique saturé, linéaire ou ramifié, éventuellement cyclique, comprenant entre 4 et 20 atomes de carbone, plus particulièrement entre 6 et 14 atomes de carbone. A titre d'exemples, on peut citer un butyle, un pentyle, un hexyle, un heptyle, un octyle, un décyle, un dodécyle, un tridécyle, etc.

Par groupe « alkylène », on entend un groupe aliphatique insaturé, linéaire ou ramifié, éventuellement cyclique, comprenant entre 4 et 20 atomes de carbone, plus particulièrement entre 6 et 14 atomes de carbone, et comportant une ou plusieurs doubles liaisons, notamment de 1 à 3. A titre d'exemples, on peut citer un butylène, pentylène, hexylène, heptylène, octylène, nonylène, décylène, undécylène, dodécylène, tridécylène, etc. En particulier, on peut citer un nonylène, en particulier un nonyl-2-ène, un octylène, un décylène, un dodécylène, un décyldiène, un tridécyltriène, etc.

Comme groupe alkyle substitué par un groupement -OH, on peut par exemple citer un nonanol, en particulier un nonan-2-ol.

Comme groupe alkylène substitué par un groupement -OH, on peut par exemple citer un octènol.

Plus particulièrement, le cyclolipopeptide selon l'invention est un cyclolipopeptide de formule (A), telle que mentionnée ci-dessus, dans lequel R est choisi dans le groupe constitué des groupes :
- alkyle comprenant entre 6 et 14 atomes de carbone, à l'exception des groupes nonyle et undécyle; ou
- alkylène comprenant entre 6 et 14 atomes de carbone, à l'exception des groupes undéc-4-ylène et tridéc-6-ylène; ou
- alkyle substitué par un groupement -OH et comprenant entre 6 et 14 atomes de carbone; et
- alkylène substitué par un groupement -OH et comprenant entre 6 et 14 atomes de carbone.

Plus particulièrement encore, le cyclolipopeptide selon l'invention est choisi parmi les cyclolipopeptides de formule (A), telle que définie ci-dessus, dans laquelle R est choisi dans le groupe constitué des groupes : nonylène, en particulier nonyl-2-ène, nonanol, en particulier nonan-2-ol, heptyle, octylène, octyle, décanyl, décanol, décyldiène, décylène, octènol, dodécylène et tridécyltriène.

Selon un des aspects de la présente invention, le cyclolipopeptide décrit dans la présente demande est susceptible d'être obtenu à partir d'une bactérie telle que décrite dans le cadre de la présente divulgation.

Ainsi, la présente invention concerne également un procédé de production d'un cyclolipopeptide tel que défini ci-dessus, comprenant une étape de culture d'une bactérie telle que décrite précédemment, dans des conditions permettant la production desdits cyclolipopeptides.

En particulier, la bactérie est cultivée dans des conditions qui seront adaptées par l'Homme du métier qui saura déterminer les conditions de culture, notamment la température de culture, idéales pour la production des cyclolipopeptides.

Plus particulièrement, la bactérie est cultivée pendant 24 à 96 heures, de préférence pendant 72 heures, à une température comprise entre 4 et 25°C, de préférence entre 12 et 18°C, sous agitation à une vitesse comprise entre 70 et 130 rpm, de préférence 100 rpm.

Le milieu de culture est en particulier le milieu Marine Broth (Difco®) ou un milieu synthétique appelé milieu F29 contenant un tampon phosphate 47 mM, pH 7,4 (7,6g K2HPO4, 3g KH2PO4 -Sigma®), 3% de sels marins (SeaSalts-Sigma®), 50 mM de saccharose et 20mL de MEM 50X par litre, de préférence le milieu synthétique F29.

Selon un de ces aspects, le procédé de production comprend également une étape d'isolation des cyclolipopeptides, faisant suite à l'étape de culture de la bactérie.

En particulier, cette isolation est réalisée par la récolte du surnageant de culture, par exemple par centrifugation de ce dernier.

Selon un des ces aspects, le procédé de production comprend également une étape de purification des cyclolipopeptides, faisant suite à l'étape d'isolation précédemment mentionnée.

En particulier, la purification est réalisée par toute méthode chromatographique appropriée pour l'Homme du métier, comme par exemple par une combinaison de chromatographie liquide couplée à une analyse biofonctionnelle.

Ainsi, selon un de ces aspects, le procédé de production des cyclolipopeptides selon l'invention comprend les étapes suivantes :
a) culture d'une bactérie telle que décrite dans le cadre de la présente divulgation
   dans des conditions permettant la production des cyclolipopeptides, en particulier dans du milieu de culture F29 tel que décrit dans le cadre de la présente invention ; et/ou
b) isolation des cyclolipopeptides ; et/ou
c) purification des cyclolipopeptides.

Les cyclolipopeptides selon l'invention présentent une activité puissante et sélective des bactéries à Gram-négatif.

Ainsi, la présente invention concerne également au moins un cyclolipopeptide selon l'invention, pour son utilisation en tant que médicament. Plus particulièrement, l'invention concerne un cyclolipopeptide selon l'invention, pour son utilisation en tant que médicament.

Selon un de ces aspects, l'invention concerne également au moins un cyclolipopeptide selon l'invention, pour son utilisation en tant qu'agent antimicrobien. Plus particulièrement, l'invention concerne un cyclolipopeptide selon l'invention, pour son utilisation en tant qu'agent antimicrobien.

En particulier, le(s)dit(s) cyclolipopeptide(s) est(sont) utilisé(s) pour le nettoyage de la peau, plus particulièrement pour le lavage des mains, ou comme agent actif dans des produits d'hygiène, plus particulièrement des produits d'hygiène corporelle.

La présente divulgation, selon un autre de ces aspects, concerne également une méthode de traitement qui comprend l'administration, d'une dose efficace d'au moins un cyclolipopeptide selon l'invention, en particulier d'un cyclolipopeptide selon l'invention. Ladite méthode de traitement est en particulier une méthode de traitement antibactérienne, notamment pour le nettoyage de la peau, pour le lavage des mains, ou pour l'hygiène corporelle.

Selon un autre de ces aspects, la présente invention concerne également l'utilisation d'au moins un cyclolipopeptide selon l'invention, en tant qu'agent phytosanitaire, en particulier pour la protection des plantes, des champignons et des insectes. Plus particulièrement, l'invention concerne l'utilisation d'un cyclolipopeptide selon en tant qu'agent phytosanitaire, en particulier pour la protection des plantes, des champignons et des insectes. La présente invention, selon un autre de ces aspects, concerne également une méthode de traitement phytosanitaire, qui comprend l'administration d'une dose efficace d'au moins un cyclolipopeptide selon l'invention, en particulier d'un cyclolipopeptide selon l'invention, en particulier à une plante, un champignon ou un insecte.

Selon encore un autre de ces aspects, la présente invention concerne également l'utilisation d'au moins un cyclolipopeptide selon l'invention, en tant qu'agent conservateur pour l'industrie agroalimentaire ou l'industrie cosmétique.

Selon un autre de ces aspects, la présente invention concerne une méthode pour améliorer la conservation de produit(s) à destination de l'industrie agroalimentaire ou cosmétiques, comprenant l'adjonction d'au moins un cyclolipopeptide selon l'invention au(x) produit(s) à conserver.

Selon encore un autre de ces aspects, la présente invention concerne une composition cosmétique et/ou dermatologique comprenant au moins un cyclolipopeptide selon l'invention dans un véhicule physiologiquement acceptable. De préférence, lesdites compositions comprennent un cyclolipopeptide selon l'invention. Plus particulièrement, le(s)dit(s) cyclolipopeptide est(sont) combiné(s) avec un adjuvant ou un solvant.

On entend par « composition cosmétique » une substance ou une préparation destinée à être mise en contact avec les diverses parties superficielles du corps humain, notamment l'épiderme, les systèmes pileux et capillaire, les ongles, les lèvres et les organes génitaux externes, ou avec les dents et les muqueuses buccales, en vue, exclusivement ou principalement, de les nettoyer, de les embellir, de les parfumer, d'en modifier l'aspect, de les protéger, de les maintenir en bon état ou de corriger les odeurs corporelles.

On entend par « composition dermatologique » une substance ou préparation destinée à être mise en contact avec les diverses parties superficielles du corps humain, notamment l'épiderme, les systèmes pileux et capillaire, les ongles, les lèvres et les organes génitaux externes, ou avec les dents et les muqueuses buccales, en vue de la prévention et/ou du traitement de pathologies de la peau. Une composition dermatologique est destinée à une utilisation thérapeutique.

La présente invention concerne également une composition pharmaceutique comprenant au moins un cyclolipopeptide selon l'invention dans un véhicule pharmaceutiquement acceptable. De préférence, ladite composition comprend un cyclolipopeptide selon l'invention. Plus particulièrement, le(s)dit(s) cyclolipopeptide(s) est(sont) combiné(s) avec un adjuvant ou un solvant.

Ainsi, un cyclolipopeptide selon la présente invention peut être utilisé aussi bien dans des applications destinées à l'alimentation, plus précisément dans le domaine de la conservation des aliments, que dans des applications destinées à l'être humain, comme médicament, ou comme agent dermatologique permettant d'éliminer les agents microbiens, les bactéries, notamment celles présentes à la surface de la peau, et plus particulièrement sur les mains. Un cyclolipopeptide selon la présente invention peut également être utilisé dans le domaine cosmétique. Il peut être par ailleurs utilisé dans des applications phytosanitaires.

Les excipients pharmaceutiquement acceptables sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, les cyclolipopeptides tels que définis ci-dessus, peuvent être administrés sous forme de doses unitaires, en mélange avec des excipients pharmaceutiques classiques, aux animaux et aux êtres humains.

Les formes d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, parentérale telle que transdermique, sous cutanée, intramusculaire ou intraveineuse, les formes d'administration rectale et les implants. Pour l'application topique, on peut utiliser les cyclolipopeptides selon l'invention dans des crèmes, gels, pommades ou lotions.

Lorsqu'une composition solide sous forme de comprimés est préparée, le cyclolipopeptide peut être mélangé avec un véhicule pharmaceutique, tel que la gélatine, l'amidon, le lactose, le stéarate de magnésium, le talc, la gomme arabique ou analogues.

Les comprimés peuvent également être enrobés avec du saccharose, un dérivé cellulosique, ou d'autres matières appropriées ou encore ils peuvent être traités de telle sorte qu'ils aient une activité prolongée ou retardée et qu'ils libèrent d'une façon continue une quantité prédéterminée de principe actif.

Une préparation sous forme de gélules peut par exemple être obtenue en mélangeant l'ingrédient actif avec un diluant et en versant le mélange obtenu dans des gélules molles ou dures.

Les compositions pharmaceutiques contenant un cyclolipopeptide conformément à l'invention, peuvent aussi se présenter sous forme liquide, par exemple, sous forme de solutions, d'émulsions, de suspensions ou de sirops, et notamment sous une forme adaptée pour une administration orale ou intranasale, par exemple. Les supports liquides appropriés peuvent être, par exemple, de l'eau, des solvants organiques tels que le glycérol ou les glycols, de même que leurs mélanges, dans des proportions variées, dans l'eau.

Une préparation sous forme de sirop ou d'élixir ou pour l'administration sous forme de gouttes peut également contenir l'ingrédient actif conjointement avec un édulcorant, acalorique par exemple, ainsi qu'un agent donnant du goût et un colorant approprié.

Les poudres ou les granules dispersibles dans l'eau peuvent par exemple contenir l'ingrédient actif en mélange avec des agents de dispersion ou des agents mouillants, ou des agents de mise en suspension, comme la polyvinylpyrrolidone, de même qu'avec des édulcorants ou des correcteurs de goût.

La dose administrée par jour à chaque patient, humain ou animal, sera déterminée, selon la pratique habituelle, par le médecin ou le vétérinaire selon le mode d'administration, le poids et la réponse dudit patient. En général, la dose journalière du cyclolipopeptide selon l'invention sera la dose efficace la plus faible de cyclolipopeptide capable de produire l'effet thérapeutique recherché.

Par « dose efficace », on désigne toute quantité d'une composition qui améliore un ou plusieurs des paramètres d'efficacité recherchée.

Il peut y avoir des cas particuliers où des dosages plus élevés ou plus faibles sont appropriés; de tels dosages ne sortent pas du cadre de la présente invention.

Dans les compositions cosmétiques ou dermatologiques selon l'invention, les excipients physiologiquement acceptables sont choisis selon la forme cosmétique ou dermatologique et le mode d'administration souhaité, parmi les excipients habituels qui sont connus de l'Homme du métier.

Un milieu physiologiquement acceptable est un milieu non toxique et susceptible d'être appliqué sur la peau et les phanères des êtres humains et d'aspect, d'odeur et de toucher agréables.

Pour une administration par voie orale, la composition cosmétique ou dermatologique selon l'invention peut se présenter sous toute forme adaptée, en particulier sous forme d'une solution buvable, d'un comprimé, d'une gélule, d'une capsule ou encore d'un aliment ou complément nutritionnel.

Pour une application topique sur la peau, la composition peut avoir la forme notamment de solutions aqueuses ou huileuses ou de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique ou de mousses. Ces compositions sont préparées selon les méthodes usuelles.

Les quantités de cyclolipopeptides dans les compositions cosmétiques ou dermatologiques selon l'invention seront comprises entre 0 ,1% et 3%, de préférence entre 0,2% et 0,5%.

Les compositions cosmétiques ou dermatologiques selon l'invention peuvent par exemple constituer des crèmes de nettoyage, de protection, de traitement ou de soin pour le visage, pour les mains, pour les pieds, pour les grands plis anatomiques ou pour le corps (par exemple crèmes de jour, crèmes de nuit), des laits corporels de protection ou de soin, des lotions, gels ou mousses pour le soin de la peau, comme des lotions de nettoyage, des compositions désodorisantes.

Les compositions peuvent également consister en des préparations solides constituant des savons ou des pains de nettoyage.

Les compositions peuvent aussi être conditionnées sous forme de composition pour aérosol comprenant également un agent propulseur sous pression.

En particulier, la composition dermatologique ou cosmétique selon l'invention peut être choisie parmi les déodorants ou les laits ou gels moussant nettoyants. Les figures et exemples suivants illustrent la présente invention sans en limiter la portée.

### Figures

**Figure 1** : Protocole expérimental mis en place pour évaluer les effets de la souche *hCg-6* sur la survie du naissain d'huître.
**Figure 2** : Evolution de la mortalité d'huîtres diploïdes naïves en présence d'un pathogène (*Vibrio splendidus* LGP32) sans (●) ou avec balnéation préalable avec la souche *Pseudoalteromonas hCg-6* (■) et *Pseudoalteromonas prydzensis* (▲).
**Figure 3** : Evaluation du pouvoir nettoyant antibactérien des cyclolipopeptides selon l'invention par le test RAPA :
   - (A) : Boîte témoin, non traitée et non ensemencée,
   - (B) et (C) : Boîtes non traitées par l'échantillon et ensemencées respectivement avec *E. coli* ATCC 25922 et *E. coli* P7,
   - (D) et (E) : Boîtes traitées par 200 µg de cyclolipopeptides selon l'invention et ensemencées respectivement par *E. coli* ATCC 25922 et *E. coli* P7.

### Exemples

### (i) Isolement et identification de la souche Pseudoalteromonas hCg-6

La souche bactérienne *hCg-6* a été isolée de l'hémolymphe de *Crassostrea gigas.*

Ces huîtres sont récoltées dans la péninsule de Rhuys dans le golf du Morbihan, France (47°30'50 Nord, 2°37'50 Ouest, système WGS84). Après une ouverture prudente des huîtres, l'hémolymphe est collectée dans la cavité péricardique en utilisant une aiguille stérile à usage unique. Pour l'isolement bactérien, chaque échantillon individuel d'hémolymphe (1,5ml) est posé directement sur de l'agar marin (Difco® 2216) à l'aide d'un ensemenseur automatique (WASP, AES Chemunex, France) et incubé pendant 72 H à 18°C.

L'étude des caractères génétiques et biochimiques de *hCg-6* permettent de définir une nouvelle espèce.

Les études de mobilité et concernant la morphologie ont été conduites en utilisant un microscope optique (Olympus BX50). La capacité de la souche bactérienne à hydrolyser le tween 40 et 80 a été testée selon la méthode de Lelliot et Stead (1987). Les activités enzymatiques et les caractéristiques biochimiques ont été déterminées en utilisant les kits suivants : API 20 E, API 50 CH et API ZYM (Biomérieux) selon les conditions indiquées par le fournisseur. Selon les indications de Park et al. (2005), les milieux fournis par Biomérieux utilisés dans les kits d'inoculation ont été complétés par 2% w/v de sels de mer (Sigma). Enfin, les analyses relatives à la constitution en acides gras ont été réalisées selon les méthodes décrites dans Kämpfer & Kroppenstedt, 1996; Kuykendall et al., 1988; Miller, 1982.

Il a ainsi pu être déterminé que cette bactérie Gram négative ne forme pas de spores, est un organisme mobile (0,6-0,9 µm de largeur, 1,9-3µm de longueur), capable de former des colonies faiblement colorées en marron.

La croissance est observée entre 4 et 30°C (croissance optimale entre 25 et 30°C).

Les acides gras principaux qui la constitue sont les suivants : 3=C16:1 ω7c/i-C15:0 2-OH (36,6%), C18:1ω7c (18,6%), C16:0 (13,8%).

Les autres caractéristiques physiologiques et biochimiques de la souche bactérienne *hCg-6* sont rassemblées dans le tableau 1 ci-dessous. En plus de ces résultats, il a également été déterminé que la souche est catalase -, oxydase +, et hydrolyse le Tween 40 et 80. Au cours de l'analyse utilisant le kit API ZYM, la souche *hCg-6* a montré une réaction positive à la phosphatase alkaline, la phosphatase acide, la naphtol-AS-BI-phosphohydrolase, l'alpha-glucosidase et la N-acetyl-beta-glucosaminidase. Une absence de réaction a été observée pour les autres activités enzymatiques inclues dans le kit API ZYM. Suite aux analyses réalisées avec le kit API 20 NE, les tests suivants sont positifs : réduction du nitrate, hydrolyse de l'esculine, assimilation du glucose, maltose, N-acétyl-glucosamine, malate et citrate. Les substances suivantes ne sont pas utilisées : D-galactose, L-rhamnose, D-sorbitol, D ou L-arabinose, D ou L-arabitol et D-melibiose. La souche bactérienne *hCg-6* utilise le glucose, le saccharose, le tréhalose, le fructose, le D-cellobiose et le D-maltose comme sources de carbone et d'énergie.

La souche est résistante à la polymyxine (50µg) et à la cefalexine mais sensible à l'enrofloxacine, la cefquinome 30, la gentamicine, l'acide oxolinique, la tétracycline et l'amocicilline.

**Tableau 1 : Caractéristiques physiologiques et biochimiques de la souche hCg-6:**

| **Caractéristiques** | ***hCg-6*** |
|---|---|
| Composition en ADN G+C (mol%) | 41,2 |
| | |
| Pigmentation | f |
| | |
| Croissance à/dans: | |
| 4°C | + |
| 37°C | - |
| 0.5% NaCl | + |
| ≥12% NaCl | - |
| Hydrolyse de l'amidon | - |
| | |
| Utilisation de : | |
| D-Glucose | + |
| D-fructose | + |
| D- Arabinose | - |
| Glycérol | - |
| Mannose | + |
| D-Mannitol | - |
| Citrate | + |
| N-Acétyl-glucosamine | + |
| | |
| Sensible à : | |
| Polymyxin (300 ED) | - |

| | |
|---|---|
| +, Positif; -, négatif ; f : faible réaction | |

Cette souche a fait l'objet d'un dépôt selon le Traité de Budapest, le 22 mai 2013, auprès de la Collection Nationale de Culture de Microorganismes (CNCM, Paris, France) sous le numéro I-4753.

### (ii) Activité de la souche Pseudoalteromonas hCg-6 en aquaculture

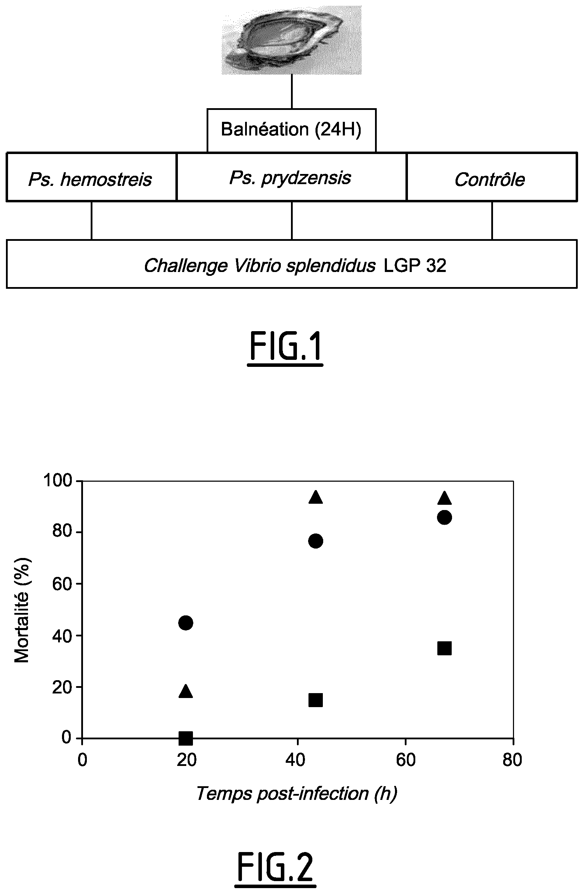

Pour évaluer le rôle de bioprotection de la souche *hCg-6,* des infections expérimentales par *Vibrio splendidus* LGP32 (injection dans le muscle adducteur) d'huitres diploïdes naïves (naissain de 18 mois) ont été réalisées avec ou sans balnéation préalable avec la souche *hCg-6.* Le protocole expérimental est présenté en Figure 1.

Les résultats montrent que la balnéation préalable des huîtres (n=19) en présence de la souche *hCg-6* se traduit par une réduction significative de la mortalité (Figure 2). En effet, après 60h, la mortalité globale est stabilisée à 35% tandis que le lot d'huîtres non balnées (n=22) avec *hCg-6* présente un taux de mortalité de 86% et de 94% pour celles (n=16) balnées avec la souche de *Pseudoalteromonas prydzensis* (contrôle).

### (iii) Production et isolation de cyclolipopeptides

Après culture de la souche *Pseudoalteromonas hCg-6* pendant 72h à 18°C sous agitation (100 rpm) dans le milieu synthétique F29 (tampon phosphate 47 mM, pH 7,4 (7,6g K2HPO4, 3g KH2PO4 -Sigma®), 3% de sels marins (SeaSalts-Sigma®), 50mM de saccharose et 20mL de MEM 50X par litre), le surnageant de culture, contenant les molécules actives, est récolté par centrifugation 30 minutes à 7500 rpm à 4°C. Une combinaison de chromatographies liquides couplée à une analyse biofonctionnelle est mise en place afin de purifier les composés actifs présents dans le surnageant de culture. Deux solvants sont utilisés : H2O milliQ 0,07% TFA (Sigma®) (solvant A) et l'acétonitrile (Carlo Erba®) 0.07% TFA (solvant B). Le surnageant de culture est déposé sur une colonne SPE (Upti Clean columns C18-S-Interchim®) préalablement conditionnée suivant les instructions du fournisseur. Après un lavage de la colonne par un mélange des solvants A et B (90/10), l'élution est assurée par un mélange des solvants A et B 60-40 (v/v).

La fraction éluée est ensuite analysée en chromatographie en phase inverse sur une colonne HTec (250 mm X 4.6 mm -Macherey-Nagel®). L'élution est suivie à 220 et 280 nm et assurée par un gradient linéaire biphasique de solvant B (20 à 28% en 8 min puis 28 à 33% en 15 min) à un débit de 0,8mL.min-1 à 40°C. Les pics d'absorbances sont collectés manuellement, lyophilisés, testés pour leur activité antibactérienne et stockées à -20°C. Une série de 11 composés bioactifs présentant des temps de rétention différents a été ainsi purifiée et analysée par spectrométrie de masse (MALDI TOF/TOF Autoflex III smartbeam (Bruker Daltonics), matrice utilisée : HCCA, méthode low mass ou LIFT).
Les masses moléculaires des ions moléculaires (M+H+) des composés actifs sont les suivants : 927, 971, 941, 953, 939, 967, 965, 985, 995, 1005 et 968.

### (iv) Caractérisation structurale des cyclolipopeptides

La caractérisation structurale des 11 composés bioactifs a été menée par résonance magnétique nucléaire (RMN) et Spectrométrie de masse (Maldi-TOF/TOF).

Les échantillons analysés par RMN contiennent environ 1 mM de peptide dissous dans l'eau (90% H20 et 10% D2O), à pH 5. Les spectres RMN ont été enregistrés à 298K sur un spectromètre RMN Bruker Avance 500, équipé d'une cryosonde TXI 5 mm triple résonance (1H, 13C, 15N).

L'attribution des déplacements chimiques a été effectuée à l'aide de séquences standards (Bruker) de spectres 1D et 2D homonucléaires et hétéronucléaires : TOCSY, NOESY, 13C-HSQC et 13C-HMBC. Un délai de relaxation de 1,4 s a été utilisé pour toutes les expériences et des temps de mélange de 100 ms et 250 ms pour les expériences TOCSY et NOESY, respectivement. Des tailles de matrices de 2K ou 4K en F2 et de 320K à 400K dans la dimension F1 ont été utilisées. De 8 à 32 scans ont été accumulés pour chaque valeur de t1.

Les déplacements chimiques sont calibrés au travers du déplacement chimique résiduel de l'eau. Le logiciel TOPSPIN a été utilisé pour traiter les spectres obtenus.

Les analyses ont montré que les 11 composés sont de nature lipopeptidique. Ils sont constitués d'un cycle heptapeptidique acylé par une chaîne hydrocarbonée variant en longueur (8 à 14 atome de carbone), en niveau d'insaturation et en hydroxylation, comme indiqué dans le tableau 2 ci-dessous.

**Tableau 2 : Structure des cyclolipopeptides produits par la souche Pseudoalteromonas hCg-6**

| Structure générale des cyclolipopeptides | N° | R |
|---|---|---|
| 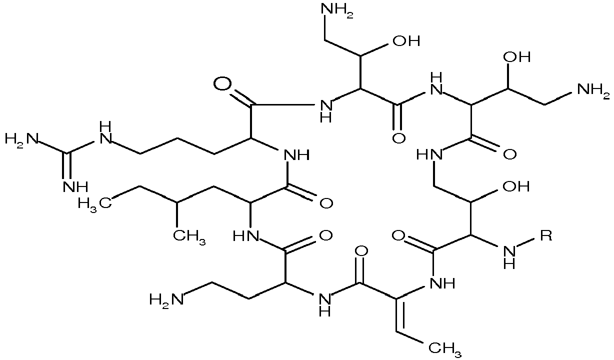 | 1 | C10 :1* |
| | 2 | C10 :0-OH** |
| | 3 | C8 :0 |
| | 4 | C9 :1 |
| | 5 | C9 :0 |
| | 6 | C11 :2 |
| | 7 | C11 :1 |
| | 8 | C11 :0-OH** |
| | 9 | C13 :1 |
| | 10 | C14 : 3 |
| | 11 | C11 :0 |

| | | |
|---|---|---|
| * la double liaison est en position 3, configuration cis ** le groupement OH est en position 3 | | |

### (v) Mécanisme d'action des cyclolipopeptides

Le cyclolipopeptide n°2 a été testé sur une collection de souches de *Vibrio splendidus* isolés d'huître. Il présente des concentrations minimales inhibitrices de 1,56 à 50 µM. Les CMI ont été définies en milieu liquide en suivant le protocole de Wiegand, Hilpert, and Hancock 2008.

Dans un premier temps une perte de cultivabilité du pathogène d'huître *V*. *splendidus* LGP32 en présence du cyclolipopeptide a été observée, suggérant un effet bactéricide. Par des analyses en cytométrie de flux (FacsCalibur), l'activité bactéricide sur les bactéries a été confirmée en utilisant un double-marquage au Syto9 et à l'iodure de propidium (indicateur de perméabilisation des membranes bactériennes).

Le détail du mécanisme d'action a été révélé par cytométrie en flux au moyen d'autres marqueurs fluorescents d'état physiologique. Ainsi, la perte de cultivabilité de la souche LGP32 exposée au cyclolipopeptide corrèle avec un arrêt de la respiration membranaire (révélée par marquage au CTC) et la dépolarisation de la membrane cytoplasmique (qui devient perméable au diBAC). Par ailleurs, le nombre de cellules totales de LGP32 (révélé par marquage au SyberGreen I) diminue avec l'exposition au cyclolipopeptide, évoquant un effet lytique.

La capacité des cyclolipopeptides selon l'invention à lier le lipopolysaccharide (LPS) bactérien a été mesurée à l'aide du test chromogénique « limulus amebocyte lysate assay ». Les cyclolipopeptides selon l'invention ont démontré une forte affinité pour le LPS de *E.coli.* L'affinité des cyclolipopeptides selon l'invention pour le LPS de *E.coli* a été quantifiée par thermophorèse à microéchelle, la constante de dissociation apparente a été estimée à 10,4 µM +/- 680 nM.

### (vi) Activité biologique des cyclolipopeptides

La concentration minimale inhibitrice (CMI) des composés n°1, 2, 3, 5, 7 et 8 a été évaluée en milieu liquide en suivant le protocole de Wiegand, Hilpert, and Hancock 2008. Le spectre de bactéries cibles a été élargi à certain pathogènes humains tel que les bactéries *Salmonella enterica,* une souche clinique d'*Escherichia coli* présentant une activité beta-3-lactamase à spectre étendue et *Pseudomonas aeruginosa.* Après 24h d'incubation à température optimale, les CMI sont évaluées visuellement.

Les résultats montrent que les cyclolipopeptides présentent une activité spécifiquement dirigée contre les bactéries à Gram négatif (Tableau 3). Les CMI obtenues sont de l'ordre du µM.

**Tableau 3 : Spectre d'activité des cyclolipopeptides n°1, 2, 3, 5, 7 et 8 (CMI)**

| | | *3* | *5* | *1* | *2* | *7* | *8* | *Polymyxine B* |
|---|---|---|---|---|---|---|---|---|
| *Lactococcus garviae* | ATCC 43921 | ND | ND | - | - | ND | ND | ND |
| *Listeria monocytogenes* | SOR200 | ND | ND | - | - | ND | ND | ND |
| *Staphylococcus aureus* | ATCC 25923 | ND | ND | - | - | ND | ND | ND |
| *A. caviae* | CIP 7616 | ND | ND | ND | 17,6 | ND | ND | 3,1 |
| *A. hydrophila* | CIP 7614 | - | 25 | ND | ND | - | - | 0,8 |
| *E.coli β résistante* | P7 (souche clinique) | ND | ND | 3.1 | 8,8 | ND | ND | 1,6 |
| *E.coli* | ML35 | 100 | 50 | 12.5 | 35 | - | - | 3,1 |
| *E.coli* | SBS363 | 50 | 25 | 0,2 | 0,1 | 100 | - | 0,2 |
| *Pseudomonas aeruginosa* | ATCC 27853 | - | - | - | 35,2 | 100 | - | 1,6 |
| *S.enterica* | CIP 8297 | 12,5 | 6,3 | 0.7 | 1,4 | 25 | 100 | 0,8 |
| *V. harveyi* | ORM4 | 50 | 6,3 | 12,5 | ND | 50 | 50 | 25 |
| *V. splendidus* | LGP 32 | 50 | 6,3 | 12.5 | 4.4 | 50 | 50 | 1.6 |
| *Y.ruckeri* | ATCC 29473 | 50 | 6,3 | 0,4 | 1,1 | 0,4 | 50 | 0,4 |

Les résultats obtenus avec les souches de *E.coli* ML35 et SBS363 suggèrent un rôle des composants de la membrane externe dans la sensibilité aux cyclolipopeptides. En effet, la souche SBS363 expose un LPS à chaîne courte par rapport à celui de la souche ML35. Dans tous les cas, cette modification structurale se traduit par augmentation de l'activité antibactérienne des cyclolipopeptides.

Les CMIs définies sont comparables à celles de la polymyxine B encore appelée colistine.

L'activité cytotoxique des cyclolipopeptides selon l'invention a également été évaluée par le test au MTT (sel de tétrazolium MTT) sur la lignée cellulaire de fibroblaste 3T3. Les résultats montrent une augmentation dose-dépendante de la cytotoxicité. Néanmoins, elle reste inférieure à 50 % pour des concentrations voisines de 200 µM.

### (vii) Activité des cyclolipopeptides en dermatologie

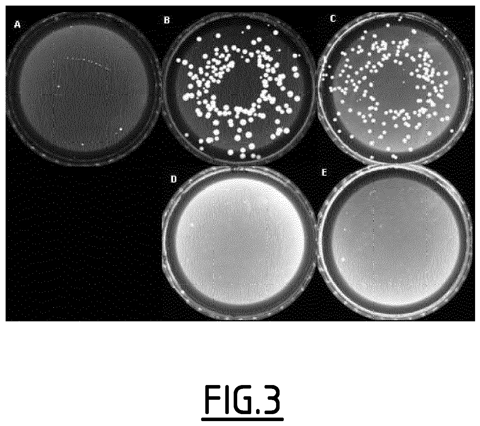

Le pouvoir antibactérien des cyclolipopeptides selon l'invention a été évalué par le test RAPA (Ansari et al, Int. J.Cosmetic Sci, 2010, 33 :107- 10). Pour simuler le lavage des mains, des boîtes de Pétri contenant du Trytone Soy Agar (TSA) sont utilisées. Le TSA est lavé manuellement avec l'échantillon (45 secondes) puis rincé à l'eau et séché à l'air. Les bactéries cibles (200 UFC) sont par la suite déposées. Les boîtes sont incubées à température optimale de croissance. Les résultats montrent que les cyclolipopeptides selon l'invention possèdent un réel pouvoir nettoyant antibactérien (Figure 3).

## Revendications

1. Cyclolipopeptide de formule A : 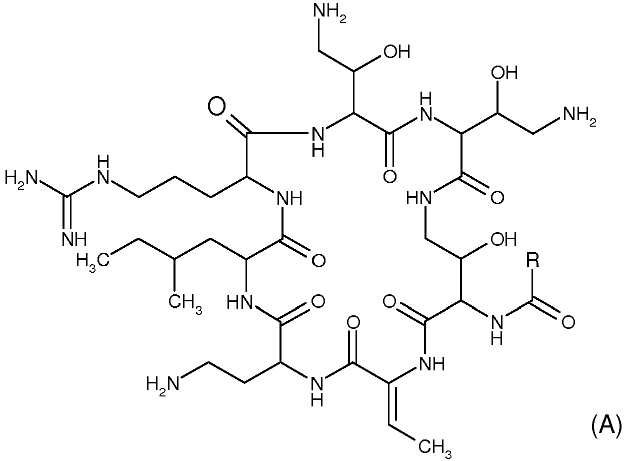 dans laquelle :
R est choisi dans le groupe constitué des groupes :
- alkyle comprenant entre 4 et 20 atomes de carbone, à l'exception des groupes nonyle et undécyle; ou
- alkylène comprenant entre 4 et 20 atomes de carbone, à l'exception des groupes undéc-4-ylène et tridéc-6-ylène; ou
- alkyle substitué par un groupement -OH et comprenant entre 4 et 20 atomes de carbone; et
- alkylène substitué par un groupement -OH et comprenant entre 4 et 20 atomes de carbone.

2. Cyclolipopeptide selon la revendication 1, dans lequel R est choisi dans le groupe constitué des groupes : nonylène, en particulier nonyl-2-ène, nonanol, en particulier nonan-2-ol, heptyle, octylène, octyle, décyldiène, décanyle, décanol, décylène, octènol, dodécylène et tridécyltriène.

3. Procédé de production d'un cyclolipopeptide tel que défini selon l'une quelconque des revendications 1 à 2, comprenant une étape de culture d'une bactérie isolée *Pseudoalteromonas hCg-6*;dans des conditions permettant la production desdits cyclolipopeptides.

4. Procédé selon la revendication 3, dans lequel la bactérie isolée est *Pseudoalteromonas hCg-6,* souche déposée selon le Traité de Budapest, le 22 mai 2013, auprès de la Collection Nationale de Culture de Microorganismes (CNCM, Paris, France) sous le numéro I-4753.

5. Procédé selon la revendication 3 ou 4, dans lequel l'étape de culture est mise en oeuvre dans du milieu F29 contenant un tampon phosphate 47 mM, pH 7.4, 3% de sels marins, 50 mM de saccharose et 20 ml de MEM 50X par litre.

6. Composition cosmétique et/ou dermatologique comprenant au moins un cyclolipopeptide selon l'une quelconque des revendications 1 à 2 dans un véhicule physiologiquement acceptable.

7. Composition pharmaceutique comprenant au moins un cyclolipopeptide selon l'une quelconque des revendications 1 à 2 dans un véhicule pharmaceutiquement acceptable.

8. Cyclolipopeptide selon l'une quelconque des revendications 1 à 2, pour son utilisation en tant que médicament.

9. Cyclolipopeptide selon l'une quelconque des revendications 1 à 2, destiné à être utilisé en tant qu'agent antimicrobien.

10. Cyclolipopeptide pour son utilisation selon la revendication 9, pour le nettoyage de la peau, en particulier pour le lavage des mains ou comme agent actif dans des produits d'hygiène, en particulier des produits d'hygiène corporelle.

11. Utilisation d'au moins un cyclolipopeptide selon l'une quelconque des revendications 1 à 2, en tant qu'agent conservateur pour l'industrie agroalimentaire ou l'industrie cosmétique.

12. Utilisation d'au moins un cyclolipopeptide selon l'une quelconque des revendications 1 à 2, en tant qu'agent phytosanitaire.

## Patentansprüche

1. Cyclolipopeptid der Formel A 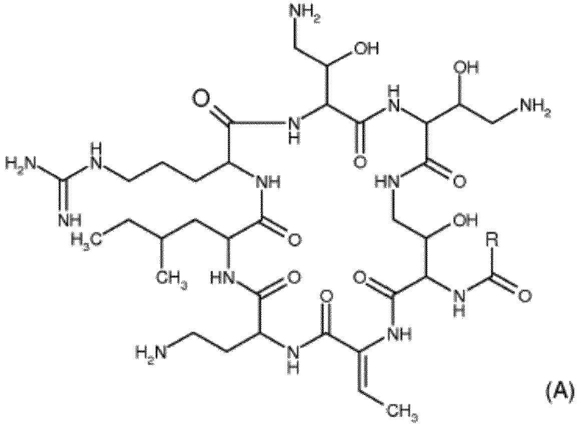 worin:
R ausgewählt ist aus der Gruppe bestehend aus den Gruppen:
- Alkyl mit 4 bis 20 Kohlenstoffatomen, mit Ausnahme von den Nonyl- und Undecylgruppen; oder
- Alkylen mit 4 bis 20 Kohlenstoffatomen, mit Ausnahme der Undec-4-ylen- und Tridec-6-ylen-Gruppen; oder
- Alkyl, die mit einer -OH-Gruppe substituiert ist, und die zwischen 4 und 20 Kohlenstoffatome aufweist; und
- Alkylen, die mit einer -OH-Gruppe substituiert ist, und die zwischen 4 und 20 Kohlenstoffatome aufweist.

2. Cyclolipopeptid nach Anspruch 1, wobei R ausgewählt ist aus der Gruppe bestehend aus den Gruppen: Nonylen, insbesondere Nonyl-2-en, Nonanol, insbesondere Nonan-2-ol, Heptyl, Octylen, Octyl, Decyldien, Decanyl, Decanol, Decylen, Octenol, Dodecylen und Tridecyltrien.

3. Verfahren zum Herstellen eines Cyclolipopeptids nach einem der Ansprüche 1 bis 2, umfassend einen Schritt des Kultivierens eines isolierten Bakteriums *Pseudoalteromonas hCg-6* unter Bedingungen, die die Herstellung der Cyclolipopeptide ermöglichen.

4. Verfahren nach Anspruch 3, wobei das isolierte Bakterium *Pseudoalteromonas hCg-6* ist, das unter der Nummer 1-4753 nach dem Budapester Vertrag, am 22. Mai 2013, in der Collection Nationale de Culture de Microorganismes (CNCM, Paris, Frankreich) als Stamm hinterlegt ist.

5. Verfahren nach Anspruch 3 oder 4, wobei der Schritt des Kultivierens in F29-Medium mit 47 mM Phosphatpuffer, pH 7,4, 3% Seesalze, 50 mM Saccharose und 20 ml MEM 50X pro Liter durchgeführt wird.

6. Kosmetische und / oder dermatologische Zusammensetzung, umfassend mindestens ein Cyclolipopeptid nach einem der Ansprüche 1 bis 2 in einem physiologisch verträglichen Träger.

7. Pharmazeutische Zusammensetzung, umfassend mindestens ein Cyclolipopeptid nach einem der Ansprüche 1 bis 2 in einem pharmazeutisch verträglichen Träger.

8. Cycolipopeptid nach einem der Ansprüche 1 bis 2 zur Verwendung als Medikament.

9. Cyclolipopeptid nach einem der Ansprüche 1 bis 2 zur Verwendung als antimikrobielles Mittel.

10. Cyclolipopeptid zur Verwendung nach Anspruch 9 zur Reinigung der Haut, insbesondere zum Händewaschen oder als Wirkstoff in Hygieneprodukten, insbesondere Körperpflegeprodukten.

11. Verwendung von mindestens einem Cyclolipopeptid nach einem der Ansprüche 1 bis 2 als Konservierungsmittel für die Lebensmittelindustrie oder die Kosmetikindustrie.

12. Verwendung von mindestens einem Cyclolipopeptid nach einem der Ansprüche 1 bis 2 als phytosanitäres Mittel.

## Claims

1. A cyclolipopeptide of formula A: 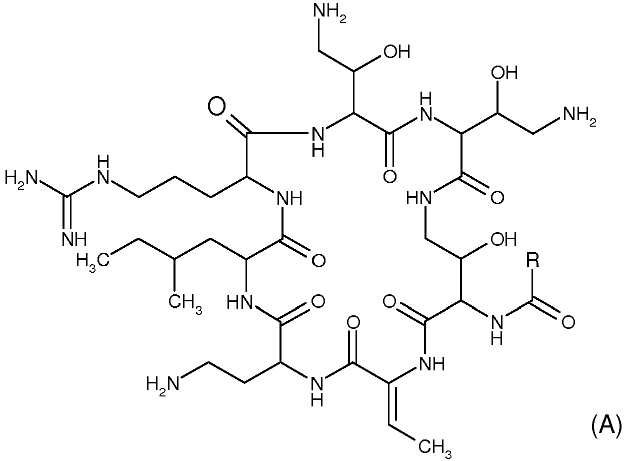 wherein:
R is selected from the group consisting of:
- alkyl groups comprising between 4 and 20 carbon atoms, except nonyl and undecyl groups; or
- alkylene groups comprising between 4 and 20 carbon atoms, except undec-4-ylene and tridec-6-ylene groups; or
- alkyl groups substituted with an -OH group and comprising between 4 and 20 carbon atoms; and
- alkylene groups substituted with an -OH group and comprising between 4 and 20 carbon atoms.

2. The cyclolipopeptide according to claim 1, wherein R is selected from the group consisting of the groups: nonylene, in particular nonyl-2-ene, nonanol, in particular nonan-2-ol, heptyl, octylene, octyl, decyldiene, decanyl, decanol, decylene, octenol, dodecylene and tridecyltriene.

3. A method for producing a cyclolipopeptide as defined according to any of claims 1 to 2, comprising a step for cultivation of an isolated bacterium *Pseudoalteromonas hCg-6*, under conditions allowing production of said cyclolipopeptides.

4. The method according to claim 3, wherein said bacterium is *Pseudoalteromonas hCg-6,* a strain deposited according to the Budapest Treaty, on May 22^{nd}, 2013, at the Collection Nationale de Culture de Microorganismes (CNCM, Paris, France) under the number I-4753.

5. The method according to claim 3 or 4 wherein the cultivation step is carried out in a synthetic medium called medium F29 containing a phosphate buffer 47 mM, pH 7.4, 3% of sea salts, 50 mM of saccharose and 20 ml of MEM 50X per liter.

6. A cosmetic and/or dermatological composition comprising at least one cyclolipopeptide according to any of claims 1 to 2 in a physiologically acceptable carrier.

7. A pharmaceutical composition comprising at least one cyclolipopeptide according to any of claims 1 to 2 in a pharmaceutically acceptable carrier.

8. The cyclolipopeptide according to any of claims 1 to 2, for its use as a drug.

9. The cyclolipopeptide according to any of claims 1 to 2, for its use as an antimicrobial agent.

10. The cyclolipopeptide for its use according to claim 9, for cleaning the skin, in particular for cleaning hands or as an active agent in hygiene products, in particular body hygiene products.

11. The use of at least one cyclolipopeptide according to any of claims 1 to 2, as a preservative for the agro-food industry or the cosmetic industry.

12. The use of at least one cyclolipopeptide according to any of claims 1 to 2, as a phytosanitary agent.
